# EUROPÄISCHE PATENTSCHRIFT

(11) **EP 0 936 220 B1**
(45) Veröffentlichungstag und Bekanntmachung des Hinweises auf die Patenterteilung: **09.04.2003**
(21) Anmeldenummer: 99100857.4
(22) Anmeldetag: 19.01.1999
(51) Int. Cl.: C07D 309/30

(54) **Verfahren zur katalytischen Herstellung von 3-Isochromanonen aus Phthalanen**
Method for the catalytic preparation of 3-isochromanones from phthalanes
Procédé pour la préparation catalytique des 3-isochromanones commencant des phthalanes

(30) Priorität: 28.01.1998 DE 19803076
(43) Veröffentlichungstag der Anmeldung: 18.08.1999
(73) Patentinhaber: Clariant GmbH, 65929 Frankfurt am Main (DE)
(72) Erfinder: Geissler, Holger Dr., 55116 Mainz (DE); Decker, Daniel Dr., 65835 Liederbach (DE); Gross, Peter, 65451 Kelsterbach (DE)

(56) Entgegenhaltungen:
- J. ALMENA ET. AL.: "1,2Di(lithiomethyl)benzene from Phthalan: Sequential Introduction of Two Different Electrophiles. " TETRAHEDRON, Bd. 51, Nr. 11, November 1995, Seiten 3351-64, XP002103591
- U. AZZENA ET. AL.: "Reductive Electrophilic Substitution of Phthalans and Ring Expansion to Isochroman Derivatives. " TETRAHEDRON LETTERS, Bd. 36, Nr. 44, 1995, Seiten 8123-6, XP002103592

## Beschreibung

Die vorliegende Erfindung betrifft ein neues Verfahren zur katalytischen Herstellung von 3-Isochromanonen aus Phthalanen.

3-Isochromanone sowie deren substituierte Derivate sind beispielsweise als Intermediate in der Synthese von Pharmaka und Pflanzenschutzmitteln von großem Interesse. So beschreiben die EP-A-0 278 595 und die WO 97/12864 die Verwendung von 3-Isochromanon als Intermediat in der Synthese von Fungiziden und Pestiziden.

3-Isochromanone werden aus Phthalanen nach dem Verfahren von Azzena et al. (Tetrahedron Lett. 1995, Vol. 36, Seite 8123ff), Almena et al. (Tetrahedron 1995, Vol. 51, S. 3351ff) und Churanov et al. (Vestn. Mosk. Univ., Khim 1975, Vol. 16, 338 ff.) hergestellt. Hierzu werden die entsprechenden 1,3-Dihydroisobenzofurane (Phthalane) mit Lithium und katalytischen Mengen Naphthalin zu der Dilithiumverbindung der folgenden Formel 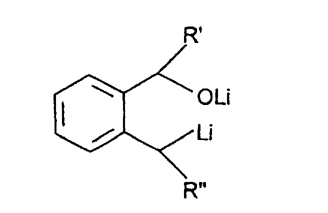 umgesetzt, die anschließend durch Reaktion mit Kohlendioxid zu dem gewünschten 3-Isochromanon weiter reagiert.
Dieses Verfahren erfordert zwei Äquivalente metallisches Lithium pro Äquivalent Produkt. Dieser hohe Verbrauch an Lithium hat zur Folge, daß eine Übertragung dieses Verfahrens auf industrielle Maßstäbe insbesondere durch die mit Umgang von metallischem Lithium verbundenen Sicherheitsvorschriften und vor allem durch die hohen Kosten unwirtschaftlich ist.

Der vorliegenden Erfindung liegt die Aufgabe zugrunde, ein neues Verfahren zur Synthese von 3-Isochromanonen bereitzustellen, das die zuvor beschriebenen Nachteile des Standes der Technik vermeidet.

Diese Aufgabe wird gelöst durch ein Verfahren zur Herstellung eines Isochroman-3-ons der Formel (I) 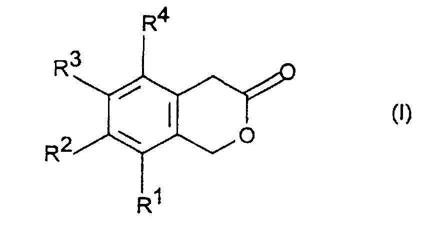 durch Umsetzung eines 1,3-Dihydroisobenzofurans der Formel (II) 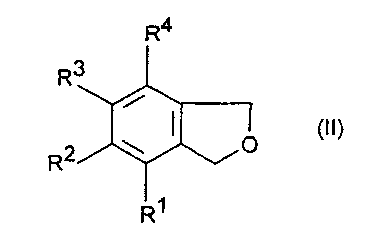 mit Kohlenmonoxid bei einem CO-Druck von 0,1 bis 20 MPa in Gegenwart eines ionischen Halogenids, eines Palladiumkatalysators und eines dipolar aprotischen Lösemittels; wobei in den Formeln (I) und (II) die Reste R¹, R², R³ und R⁴ unabhängig voneinander darstellen:
ein Wasserstoff- oder Fluoratom;
eine HO₂CCH=CH-, H₂NC(=O)-, HC(=O)NH-, CN- oder CF₃-Gruppe;
einen Alkyl-, Alkoxy- oder Acyloxyrest, mit jeweils 1 bis 18 Kohlenstoffatomen; oder einen C₆-C₁₈-Aryloxy-, Aryl- oder Heteroarylrest, wobei als Heteroatome 1 bis 3 Atome aus der Gruppe O, N und/oder S vorhanden sind;
einen R⁵C(=O)NH-, R⁵OC(=O)NH-, R⁵C(=O)R⁵N-, R⁵₂P(=O)-, R⁶₂N-, R⁶C(=O)-, R⁶OC(=O)-, R⁶OC(=O)CH=CH-, R⁷C(=O)-, R⁷₂N-, R⁷OC(=O)CH=CH- oder R⁷₂P(=O)-Rest; worin R⁵ einen C₁-C₄-Alkylrest,
R⁶ einen C₁-C₁₈-Alkylrest und R⁷ einen C₆-C₁₈-Arylrest darstellen; oder worin mindestens einer der Reste R¹, R², R³ und R⁴ durch einen Rest R⁹ substituiert ist, wobei R⁹ die gleiche Bedeutung hat wie R¹; oder worin mindestens zwei der Reste R¹, R², R³ und R⁴ untereinander verknüpft und mindestens einen aliphatischen oder aromatischen Ring mit 5 bis 18 C-Atomen bilden.

R¹, R², R³ und R⁴ bedeuten vorzugsweise Wasserstoff, Fluor, Methyl, Ethyl, Methoxy oder Ethoxy.

Das ionische Halogenid wird vorzugsweise in einer Menge von 1 bis 50 mol-%, insbesondere 2 bis 10 mol-%, bezogen auf die Verbindung der Formel (II), eingesetzt. Der Palladiumkatalysator wird vorzugsweise in einer Menge von 0,0001 bis 1 mol-%, insbesondere 0,01 bis 0,5 mol-%, bezogen auf die Verbindung der Formel (II), eingesetzt.

Als dipolar aprotisches Lösemittel können solche aus der Gruppe Dioxan, Tetrahydrofuran, N-(C₁-C₁₈-Alkyl)pyrrolidon, Ethylenglykoldimethylether, Essigsäureethylester, Acetonitril, tert.-Butylmethylether, Dibutylether, Sulfolan und N,N-Dimethylacetamid ausgewählt sein. Besonders bevorzugt sind N-Methylpyrrolidon, N-Octylpyrrolidon, N-Dodecylpyrrolidon und N,N-Dimethylacetamid.
Diese Lösemittel können sowohl in reiner Form als auch in Produkt enthaltender bzw. mit Produkt gesättigter Form eingesetzt werden. Letztere Variante wird bevorzugt bei einem kontinuierlichen Verfahren angewandt.
Bei der Verwendung von hochsiedenden Lösemitteln, wie N-Octylpyrrolidon oder N-Dodecylpyrrolidon, hat man bei der Darstellung von niedriger als das Lösemittel siedenden 3-Isochromanonen, wie z.B. dem reinen 3-lsochromanon, den Vorteil, daß das Produkt und nicht umgesetztes Startmaterial direkt aus dem Reaktionsmedium abdestilliert werden kann. Bei der Verwendung von geträgerten Palladium-Katalysatoren hat es sich als Vorteil erwiesen, den Katalysator vorher durch Filtration abzutrennen. Der Destillationssumpf kann für weitere Reaktionen wieder eingesetzt werden. Diese Variante eignet sich bevorzugt für eine effektive Katalysator- und Lösemittel-Recyclisierung.
Die Menge an Lösemittel ist prinzipiell beliebig, zweckmäßig sind jedoch 0,1 bis 2,5 molare Lösungen der Verbindung der Formel (II) in dem Lösemittel.

Das ionische Halogenid kann ein Alkali-, Ammonium- oder Phosphoniumhalogenid sein, wobei Halogenid die Bedeutung Chlorid,Bromid oder Iodid hat. Vorzugsweise ist das ionische Halogenid Ammoniumbromid, Lithiumbromid, Natriumbromid, Kaliumbromid, Tetrabutylphosphoniumbromid, Ammoniumchlorid, Lithiumchlorid, Natriumchlorid, Kaliumchlorid, Tetrabutylphosphoniumchlorid, Ammoniumiodid, Lithiumiodid, Natriumiodid, Kaliumiodid, Tetrabutylphosphoniumiodid oder eine Kombination davon.

Der Katalysator kann sowohl metallisches Palladium als auch eine Palladium-(II)-verbindung entweder in trägerloser Form (Katalysator in Masse) oder bevorzugt als geträgerte Substanz (auf einem Trägermaterial aufgebrachte(s) metallisches Palladium oder Palladiumverbindung) enthalten.

Als Trägermaterial eignen sich insbesondere Aktivkohle, Metalloxide, Metallsalze wie z.B. Sulfate oder Carbonate der Elemente der zweiten und dritten Hauptgruppe sowie der ersten und dritten Nebengruppe des Periodensystems der Elemente, wie beispielsweise Aluminiumoxid, Bariumsulfat, Calciumcarbonat und/oder Siliciumdioxid. Ein besonders bevorzugtes Trägermaterial ist Aktivkohle. Diese Trägermaterialien können in dem erfindungsgemäßen Verfahren sowohl einzeln als auch in gemischter Form verwendet werden.
Als Palladium-(II)-verbindung können insbesondere PdCl₂, PdBr₂ oder Pd(OAc)₂ verwendet werden.

In einer bevorzugten Ausführungsform enthält der Palladiumkatalysator zusätzlich einen Liganden, insbesondere eine Phosphinverbindung.
Besonders geeignet für das erfindungsgemäße Verfahren sind Katalysatoren, die eine Bis(phosphin)palladium-(II)-Verbindung enthalten. Diese Komplexe können entweder in handelsüblicher Form eingesetzt oder durch Reaktion einer Palladium-(II)-Verbindung, wie z.B. PdBr₂, PdCl₂ oder Palladium(II)-acetat, mit den entsprechenden Phosphinen erhalten werden. Die Phosphinverbindung kann hierbei Triphenylphosphin, Tritolylphosphin, Bis(diphenylphosphino)ethan, 1,4-Bis(diphenylphosphino)-butan oder 1,3-Bis(diphenylphosphino)-propan sein. Besonders bevorzugt ist die Verwendung von Bis(triphenylphosphin)-palladium(ll)-bromid der Formel PdBr₂[PPh₃]₂ oder Bis(triphenylphosphin)-palladium(II)-chlorid der Formel PdCl₂[PPh₃]₂ als Katalysator. Dieser Komplex kann durch Reaktion von Palladium-(II)-bromid oder Palladium-(II)-chlorid und Triphenylphosphin hergestellt werden.
Durch Verwendung von Phosphinen mit einem oder mehreren Chiralitätszentren ist es möglich, zu reinen Enantiomeren oder zu einem mit einem Enantiomer angereicherten Produkt zu gelangen.
Es ist auch möglich, Mischungen der zuvor beschriebenen Katalysatoren zu verwenden.

In einer weiteren, bevorzugten Ausführungsform findet die Umsetzung in Gegenwart einer anorganischen oder organischen Säure mit einem pKa-Wert von weniger als 5 statt, die vorzugsweise in einer Menge von 0,1 bis 5 Mol-%, besonders bevorzugt 1 bis 2,5 Mol-%, bezogen auf die Verbindung der Formel (II), vorhanden ist. Als Säure eignen sich insbesondere Schwefelsäure, Phosphorsäure, p-Toluolsulfonsäure, Hexafluorpropansäure und Trifluoressigsäure.

Es ist aber auch möglich, die Umsetzung in Gegenwart eines kationischen lonenaustauscherharzes durchzuführen. Besonders gut eignen sich hierzu Amberlyst® 15 (eingetragene Marke der Fa. Rohm und Haas; CAS-Nr. 9037-24-5) oder Nafion® (eingetragene Marke der Fa. DuPont; CAS-Nr. 31175-20-9).

Bevorzugt ist die Verwendung von Schwefelsäure.

Die erfindungsgemäße Umsetzung wird bei einem Kohlenmonoxid-Druck zwischen 0,1 und 20 MPa, vorzugsweise zwischen 1 und 10 MPa, sowie bei einer Temperatur vorzugsweise zwischen 20 und 200°C, insbesondere zwischen 50 und 150°C, durchgeführt.

Die erfindungsgemäße Reaktion läuft mit hoher Selektivität und guter Ausbeute ohne nennenswerte Bildung von Nebenprodukten ab.

### Beispiel 1 (Vergleichsbeispiel):

75,0 g Phthalan (1,3-Dihydroisobenzofuran) und 100 mg Schwefelsäure werden nacheinander in einem 200-ml-Autoklaven aus V4A Stahl unter SchutzgasAtmosphäre (Argon) vermischt. Anschließend wird Kohlenmonoxid mit einem Druck von 60 bar hinzugegeben und dann die Temperatur auf 130°C erhöht. Nach einer Reaktionszeit von 6 Stunden bei 130°C wird abgekühlt. Eine gaschromatographische Analyse zeigt, daß die Reaktionsmischung kein 3-Isochromanon enthält.
Anschließend wird 1 g Aktivkohle in den Autoklaven gegeben, die 30 Gew.-% Palladium enthält (30% Palladium auf Aktivkohle), der Autoklav verschlossen und Kohlenmonoxid mit einem Druck von 60 bar hinzugegeben. Die Temperatur wird auf 130°C erhöht. Nach einer weiteren Reaktionszeit von 6 Stunden bei 130°C wird abgekühlt. Eine erneute gaschromatographische Analyse zeigt, daß die Reaktionsmischung wiederum kein 3-Isochromanon enthält.

### Beispiel 2:

24,0 g Phthalan (1,3-Dihydroisobenzofuran), 100 ml N-Methylpyrrolidon (NMP), 1 g 30% Palladium auf Aktivkohle, 3,00 g Lithiumbromid und 100 mg Schwefelsäure werden nacheinander in einem 200-ml-Autoklaven aus V4A Stahl unter SchutzgasAtmosphäre (Argon) vermischt. Anschließend wird Kohlenmonoxid mit einem Druck von 60 bar hinzugegeben und dann die Temperatur auf 130°C erhöht. Nach einer Reaktionszeit von 6 Stunden bei 130°C, wobei man den Kohlenmonoxid-Druck durch Zupressen von Kohlenmonoxid zwischen 70 und 80 bar hält, wird abgekühlt. Eine gaschromatographische Analyse zeigt, daß die Reaktionsmischung 12,5 g 3-Isochromanon enthält, was einer Ausbeute von 43% entspricht. Die Selektivität beträgt mehr als 95%.

Zur Aufarbeitung des Produkts wird das Palladium auf Aktivkohle nach dem Abkühlen abfiltriert. Anschließend werden in einem Dünnschichtverdampfer bei 100°C und 10 mbar das Lösemittel und nicht umgesetztes Phthalan abgedampft. Das auf diese Weise zurückgewonnene Lösemittel (NMP), das nicht umgesetztes Phthalan enthält, kann für eine neue Reaktion wiederverwendet werden.
Der das 3-Isochromanon enthaltende Rückstand wird mit Wasser versetzt. Dabei scheidet sich ein Öl ab, das 3-Isochromanon und Phthalan im Verhältnis von 5:1 enthält. Dieses Öl wird mit 3 Volumenequivalenten tert.-Butylmethylether (MTBE) aufgenommen, abgetrennt und mit Natriumsulfat getrocknet. Nach dem Evaporieren des tert.-Butylmethylethers wird der Rückstand mit 2 Volumenequivalenten Hexan versetzt, wobei das Phthalan in Lösung geht und 3-lsachromanon aus der Lösung auskristallisiert. Durch Absaugen erhält man 12,0 g 3-Isochromanon mit einem Schmelzpunkt von 79 bis 80°C.
¹H NMR (300 MHz, CDCl₃): δ = 7,20-7,40 (m, 4H), 5,31 (s, 2H), 3,71 (s, 2H).

### Beispiel 3:

Analog Beispiel 1 werden 24,0 g Phthalan (1,3-Dihydroisobenzofuran), 100 ml N-Methylpyrrolidon (NMP), 1 g 30 % Palladium auf Aktivkohle, 3,00 g Lithiumbromid und 100 mg Schwefelsäure nacheinander in einem 200-ml-Autoklaven aus V4A Stahl umgesetzt.
Zur Aufarbeitung wird das Palladium auf Aktivkohle nach dem Abkühlen abfiltriert. Anschließend wird im Dünnschichtverdampfer bei 100°C und 10 mbar das Lösemittel und nicht umgesetztes Phthalan abgedampft. Anschließend werden 20 g NMP zugesetzt und erneut im Dünnschichtverdampfer bei 100°C und 10 mbar abgedampft, so daß das nicht eingedampfte Phthalan nahezu vollständig aus dem Rückstand entfernt wird. Der das 3-Isochromanon enthaltende Rückstand wird mit Wasser versetzt. Dabei scheidet sich 3-Isochromanon als Feststoff ab. Durch Absaugen erhält man 12,3 g 3-lsochromanon mit einem Schmelzpunkt von 80-81°C. Die Ausbeute beträgt 42 %, die Selektivität mehr als 95 %.

### Beispiele 4 bis 12:

Diese Beispiele werden analog zu Beispiel 2 durchgeführt. Die Edukte und deren Mengen sowie die Reaktionsergebnisse sind in Tabelle 1 wiedergegeben.

In der Tabelle 1 werden folgende Bezeichnungen verwendet:
- in der Spalte Lösemittel steht "NMP" für N-Methylpyrrolidon und "DMAC" für N,N-Dimethylacetamid;
- in der Spalte Katalysator steht "30 % Pd auf C" für Aktivkohle, die 30 Gew.-% Palladium enthält (30% Palladium auf Aktivkohle);
- in der Spalte Säure steht H₂SO₄ für 96 %ige Schwefelsäure;
- CO bezeichnet den Kohlenmonoxid-Druckbereich bei der Reaktionstemperatur von 130°C.

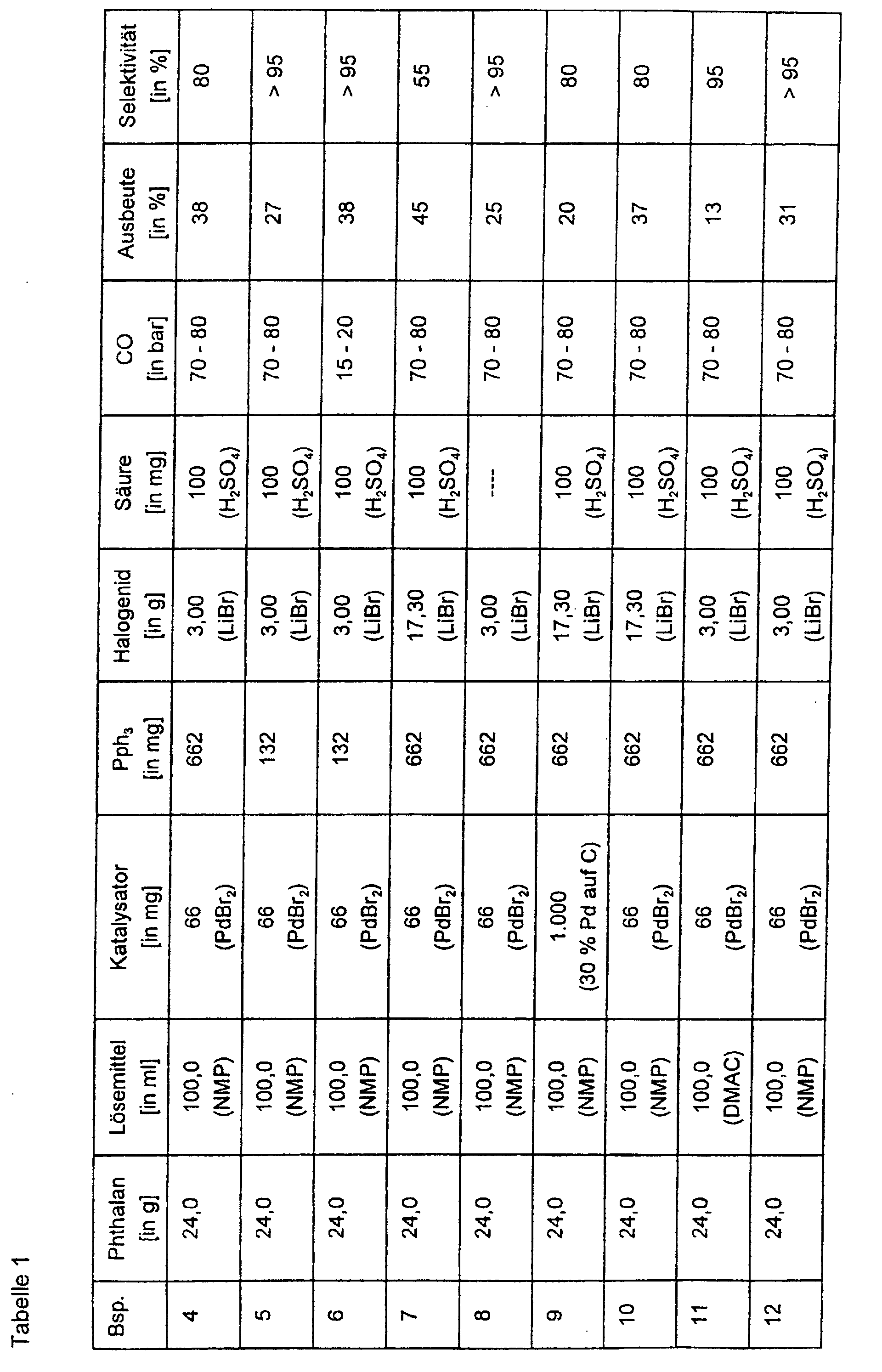

## Patentansprüche

1. Verfahren zur Herstellung eines Isochroman-3-ons der Formel (I) 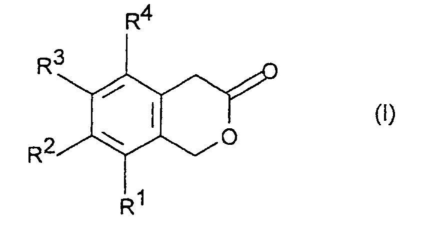 durch Umsetzung eines 1,3-Dihydroisobenzofurans der Formel (II) 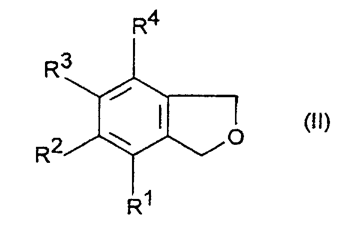 mit Kohlenmonoxid bei einem CO-Druck von 0,1 bis 20 MPa in Gegenwart eines ionischen Halogenids, eines Palladiumkatalysators und eines dipolar aprotischen Lösemittels; wobei in den Formeln (I) und (II) die Reste R¹, R², R³ und R⁴ unabhängig voneinander darstellen:
ein Wasserstoff- oder Fluoratom;
eine HO₂CCH=CH-, H₂NC(=O)-, HC(=O)NH-, CN- oder CF₃-Gruppe;
einen Alkyl-, Alkoxy- oder Acyloxyrest, mit jeweils 1 bis 18 Kohlenstoffatomen; oder
einen C₆-C₁₈-Aryloxy-, Aryl- oder Heteroarylrest, wobei als Heteroatome 1 bis 3 Atome aus der Gruppe O, N und/oder S vorhanden sind;
einen R⁵C(=O)NH-, R⁵OC(=O)NH-, R⁵C(=O)R⁵N-, R⁵₂P(=O)-, R⁶₂N-, R⁶C(=O)-, R⁶OC(=O)-, R⁶OC(=O)CH=CH-, R⁷C(=O)-, R⁷₂N-, R⁷OC(=O)CH=CH- oder R⁷₂P(=O)-Rest; worin R⁵ einen C₁-C₄-Alkylrest, R⁶ einen C₁-C₁₈-Alkylrest und R⁷ einen C₆-C₁₈-Arylrest darstellen; oder worin mindestens einer der Reste R¹, R², R³ und R⁴ durch einen Rest R⁹ substituiert ist, wobei R⁹ die gleiche Bedeutung hat wie R¹; oder worin mindestens zwei der Reste R¹, R², R³ und R⁴ untereinander verknüpft und mindestens einen aliphatischen oder aromatischen Ring mit 5 bis 18 C-Atomen bilden.

2. Verfahren nach Anspruch 1, **dadurch gekennzeichnet, daß** die Reste R¹, R², R³ und R⁴ unabhängig voneinander Wasserstoff, Fluor, Methyl, Ethyl, Methoxy oder Ethoxy bedeuten.

3. Verfahren nach Anspruch 1 oder 2, **dadurch gekennzeichnet, daß** das dipolar aprotische Lösemittel Dioxan, Tetrahydrofuran, N-(C₁-C₈-Alkyl)pyrrolidon, N-Methylpyrrolidon, Ethylenglykoldimethylether, Essigsäureethylester, Acetonitril, tert.-Butylmethylether, Dibutylether, Sulfolan oder N,N-Dimethylacetamid ist.

4. Verfahren nach mindestens einem der Ansprüche 1 bis 3, **dadurch gekennzeichnet, daß** das ionische Halogenid ein Alkali-, Ammonium- oder Phosphoniumhalogenid ist, wobei Halogenid die Bedeutung Chlorid, Bromid oder Iodid hat.

5. Verfahren nach mindestens einem der Ansprüche 1 bis 4, **dadurch gekennzeichnet, daß** das ionische Halogenid Ammoniumbromid, Lithiumbromid, Natriumbromid, Kaliumbromid, Tetrabutylphosphoniumbromid, Ammoniumchlorid, Lithiumchlorid, Natriumchlorid, Kaliumchlorid, Tetrabutylphosphoniumchlorid, Ammoniumiodid, Lithiumiodid, Natriumiodid, Kaliumiodid und/oder Tetrabutylphosphoniumiodid ist.

6. Verfahren nach mindestens einem der Ansprüche 1 bis 5, **dadurch gekennzeichnet, daß** der Palladiumkatalysator auf einem Trägermaterial aufgebrachtes metallisches Palladium enthält.

7. Verfahren nach mindestens einem der Ansprüche 1 bis 5, **dadurch gekennzeichnet, daß** der Palladiumkatalysator mindestens eine Palladium-(II)-Verbindung enthält, insbesondere PdCl₂, PdBr₂ oder Pd(OAc)₂.

8. Verfahren nach Anspruch 7, **dadurch gekennzeichnet, daß** der Palladiumkatalysator zusätzlich einen Liganden enthält, insbesondere eine Phosphinverbindung.

9. Verfahren nach Anspruch 8, **dadurch gekennzeichnet, daß** die Phosphinverbindung Triphenylphosphin, Tritolylphosphin, Bis(diphenylphosphino)ethan, 1,3-Bis(diphenylphosphino)propan oder 1,4-Bis(diphenylphosphino)butan ist.

10. Verfahren nach mindestens einem der Ansprüche 1 bis 9, **dadurch gekennzeichnet, daß** der Palladiumkatalysator Bis(Triphenylphosphino)-palladium(II)-chlorid oder Bis(Triphenylphosphino)palladium(II)-bromid enthält.

11. Verfahren nach mindestens einem der Ansprüche 1 bis 10, **dadurch gekennzeichnet, daß** die Umsetzung in Gegenwart einer anorganischen oder organischen Säure mit einem pKa-Wert von weniger als 5 durchgeführt wird.

12. Verfahren nach Anspruch 11, **dadurch gekennzeichnet, daß** die Säure Schwefelsäure, Phosphorsäure, p-Toluolsulfonsäure, Hexafluorpropansäure oder Trifluoressigsäure ist.

13. Verfahren nach mindestens einem der Ansprüche 1 bis 10, **dadurch gekennzeichnet, daß** die Umsetzung in Gegenwart eines kationischen lonenaustauscherharzes durchgeführt wird.

## Claims

1. A process for the preparation of an isochroman-3-one of the formula (I) 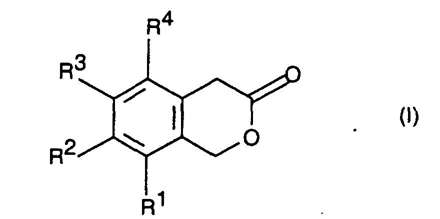 by reacting a 1,3-dihydroisobenzofuran of the formula (II) 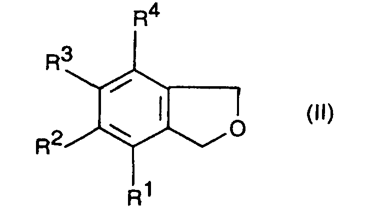 with carbon monoxide at a CO pressure of from 0.1 to 20 MPa in the presence of an ionic halide, a palladium catalyst and a dipolar aprotic solvent; where in the formulae (I) and (II) the radicals R¹, R², R³ and R⁴ independently of one another are:
a hydrogen or fluorine atom;
an HO₂CCH=CH-, H₂NC(=O)-, HC(=O)NH-, CN- or CF₃- group;
an alkyl, alkoxy or acyloxy radical having in each case 1 to 18 carbon atoms; or a C₆-C₁₈-aryloxy, aryl or heteroaryl radical, the heteroatoms being 1 to 3 atoms from the group consisting of O, N and S;
an R⁵C(=O)NH-, R⁵OC(=O)NH-, R⁵C(=O)R⁵N-, R⁵₂P(=O)-, R⁶₂N-,
R⁶C(=O)-, R⁶OC(=O)-, R⁶OC(=O)CH=CH-, R⁷C(=O)-, R⁷₂N-, R⁷OC(=O)CH=CH- or R⁷₂P(=O)- radical; in which R⁵ is a C₁-C₄-alkyl radical, R⁶ is a C₁-C₁₈-alkyl radical and R⁷ is a C₆-C₁₈-aryl radical; or in which at least one of the radicals R¹, R², R³ and R⁴ is substituted by a radical R⁹, where R⁹ has the same meaning as R¹; or in which at least two of the radicals R¹, R², R³ and R⁴ are linked to one another and form at least one aliphatic or aromatic ring having 5 to 18 carbon atoms.

2. The process as claimed in claim 1, wherein the radicals R¹, R², R³ and R⁴ independently of one another are hydrogen, fluorine, methyl, ethyl, methoxy or ethoxy.

3. The process as claimed in claim 1 or 2, wherein the dipolar aprotic solvent is dioxane, tetrahydrofuran, N-(C₁-C₈-alkyl)pyrrolidone,
N-methylpyrrolidone, ethylene glycol dimethyl ether, ethyl acetate, acetonitrile, tert-butyl methyl ether, dibutyl ether, sulfolane or N,N-dimethylacetamide.

4. The process as claimed in at least one of claims 1 to 3, wherein the ionic halide is an alkali metal, ammonium or phosphonium halide, halide being chloride, bromide or iodide.

5. The process as claimed in at least one of claims 1 to 4, wherein the ionic halide is ammonium bromide, lithium bromide, sodium bromide, potassium bromide, tetrabutylphosphonium bromide, ammonium chloride, lithium chloride, sodium chloride, potassium chloride, tetrabutylphosphonium chloride, ammonium iodide, lithium iodide, sodium iodide, potassium iodide and/or tetrabutylphosphonium iodide.

6. The process as claimed in at least one of claims 1 to 5, wherein the palladium catalyst comprises metallic palladium applied to a support.

7. The process as claimed in at least one of claims 1 to 5, wherein the palladium catalyst comprises at least one palladium(II) compound, in particular PdCl₂, PdBr₂ or Fd(OAc)₂.

8. The process as claimed in claim 7, wherein the palladium catalyst additionally comprises a ligand, in particular a phosphine compound.

9. The process as claimed in claim 8, wherein the phosphine compound is triphenylphosphine, tritolylphosphine, bis(diphenylphosphino)ethane, 1,3-bis(diphenylphosphino)propane or 1,4-bis(diphenylphosphino)butane.

10. The process as claimed in at least one of claims 1 to 9, wherein the palladium catalyst comprises bis(triphenylphosphino)palladium(II) chloride or bis(triphenylphosphino)palladium(II) bromide.

11. The process as claimed in at least one of claims 1 to 10, wherein the reaction is carried out in the presence of an inorganic or organic acid having a pKa of less than 5.

12. The process as claimed in claim 11, wherein the acid is sulfuric acid, phosphoric acid, p-toluenesulfonic acid, hexafluoropropanoic acid or trifluoroacetic acid.

13. The process as claimed in at least one of claims 1 to 10, wherein the reaction is carried out in the presence of a cationic ion exchange resin.

## Revendications

1. Procédé pour la préparation d'une isochroman-3-one de formule (I) 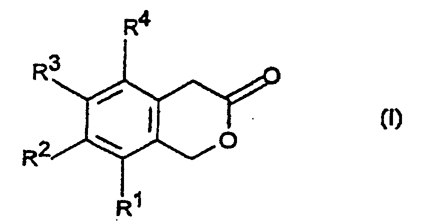 par réaction d'une 1,3-dihydroisobenzofuranne de formule (II) 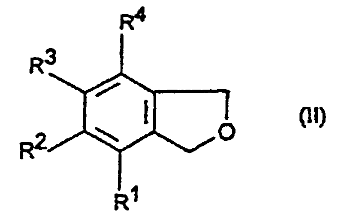 avec le monoxyde de carbone sous une pression de CO de 0,1 à 20 MPa en présence d'un halogénure ionique, d'un catalyseur au palladium et d'un solvant aprotique dipolaire ; dans les formules (I) et (II) les restes R¹, R², R³ et R⁴ représentent, indépendamment les uns des autres :
un atome d'hydrogène ou de fluor ;
un groupe HO₂CCH=CH-, H₂NC(=O)-, HC(=O)NH-, CN- ou CF₃ ;
un reste alkyle, alkoxy ou acyloxy, présentant chacun 1 à 18 atomes de carbone ; ou un reste aryloxy en C₆-C₁₈, aryle ou hétéroaryle, 1 à 3 atomes pris dans le groupe comprenant O, N et/ou S sont présents en tant qu'hétéroatomes ;
un reste R⁵C(=O)NH-, R⁵OC(=O)NH-, R⁵C(=O)R⁵N-, R⁵₂P(=O)-, R⁶₂N-, R⁶C(=O)-, R⁶OC(=O)-, R⁶OC(=O)CH=CH-, R⁷C(=O)-, R⁷₂N-, R⁷OC(=O)CH=CH- ou R⁷₂P(=O)- ; où R⁵ représente un reste alkyle C₁-C₄,
R⁶ représente un reste alkyle C₁-C₁₈ et R⁷ représente un reste alkyle en C₆-C₁₈ ; ou dans laquelle au moins un des restes R¹, R², R³ et R⁴ est substitué par un reste R⁹, R⁹ ayant la même signification que R¹ ; ou dans laquelle au moins deux des restes R¹, R², R³ et R⁴ sont reliés entre eux et forment au moins un cycle aliphatique ou aromatique présentant 5 à 18 atomes de carbone.

2. Procédé selon la revendication 1, **caractérisé en ce que** les restes R¹, R², R³ et R⁴ représentent indépendamment les uns des autres des atomes d'hydrogène, de fluor, des groupes méthyle, éthyle, méthoxy ou éthoxy.

3. Procédé selon la revendication 1 ou 2, **caractérisé en ce que** le solvant aprotique dipolaire est le dioxanne, le tétrahydrofuranne, la N-(alkyle en C₁-C₁₈)pyrrolidone, la N-méthylpyrrolidone, l'éther diméthylique d'éthylèneglycol, l'acétate d'éthyle, l'acétonitrile, l'éther tert-butylméthylique, l'éther dibutylique, la sulfolane et le N,N-diméthylacétamide.

4. Procédé selon au moins l'une des revendications 1 à 3, **caractérisé en ce que** l'halogénure ionique est un halogénure alcalin, d'ammonium ouy de phosphonium, l'halogénure représentant un chlorure, un bromure ou un iodure.

5. Procédé selon au moins l'une des revendications 1 à 4, **caractérisé en ce que** l'halogénure ionique est le bromure d'ammonium, le bromure de lithium, le bromure de sodium, le bromure de potassium, le bromure de tétrabutylphosphonium, le chlorure d'ammonium, le chlorure de lithium, le chlorure de sodium, le chlorure de potassium, le chlorure de tétrabutylphosphonium, l'iodure d'ammonium, l'iodure de lithium, l'iodure de sodium, l'iodure de potassium, l'iodure de tétrabutylphosphonium.

6. Procédé selon au moins l'un des revendications 1 à 5, **caractérisé en ce que** le catalyseur au palladium contient du palladium métal déposé sur un support.

7. Procédé selon au moins l'une des revendications 1 à 5, **caractérisé en ce que** le catalyseur au palladium contient au moins un composé de palladium(II), en particulier PdCl₂, PdBr₂ ou Pd(OAc)₂.

8. Procédé selon la revendication 7, **caractérisé en ce que** le catalyseur au palladium contient en plus un ligand, en particulier un composé de type phosphine.

9. Procédé selon la revendication 8, **caractérisé en ce que** le composé de type phosphine est la triphénylphosphine, la tritolylphosphine, le bis(diphénylphosphino)éthane, le 1,4-bis(diphénylphosphino)-propane ou le 1,4-bis(diphénylphosphino)-butane.

10. Procédé selon au moins l'une des revendications 1 à 9, **caractérisé en ce que** le catalyseur au palladium contient le chlorure de bis(triphénylphosphino)palladium(II) ou le bromure de bis (triphénylphosphino)palladium(II).

11. Procédé selon au moins l'une des revendications 1 à 10, **caractérisé en ce qu'**on met en oeuvre la réaction en présence d'un acide inorganique ou organique ayant une valeur de pKa inférieure à 5.

12. Procédé selon la revendication 11, **caractérisé en ce que** l'acide est l'acide sulfurique, l'acide phosphorique, l'acide p-toluènesulfonique, l'acide hexafluoropropanoïque et l'acide trifluoracétique.

13. Procédé selon au moins l'une des revendications 1 à 10, **caractérisé en ce qu'**on met oeuvre la réaction en présence d'une résine échangeuse d'ions cationique.
